(19) Europäisches Patentamt
European Patent Office
Office européen des brevets

(11) **EP 3 324 947 B1**

(12) **EUROPEAN PATENT SPECIFICATION**

(45) Date of publication and mention
of the grant of the patent:
**30.09.2020 Bulletin 2020/40**

(51) Int Cl.:
*A61K 9/46* *(2006.01)*      *A61K 38/00* *(2006.01)*
*A61K 9/00* *(2006.01)*      *A61K 9/16* *(2006.01)*

(21) Application number: **16742240.1**

(22) Date of filing: **19.07.2016**

(86) International application number:
**PCT/EP2016/067163**

(87) International publication number:
**WO 2017/016930 (02.02.2017 Gazette 2017/05)**

(54) **EFFERVESCENT FORMULATIONS OF ORNITHINE ASPARTATE**

SPRUDELNDE FORMULIERUNG VON ORNITHINASPARTAT

FORMULATIONS EFFERVESCENTES DE L'ORNITHINE ASPARTATE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **24.07.2015 IN 3814CH2015
24.09.2015 EP 15186592**

(43) Date of publication of application:
**30.05.2018 Bulletin 2018/22**

(73) Proprietor: **Evonik Operations GmbH
45128 Essen (DE)**

(72) Inventors:
• **JOSHI, Shraddha Sanjeev
Thane 400708
Maharashtra (IN)**
• **GUHA, Ashish Sharadchandra
Mumbai
Dombivali (E) 421204 (IN)**
• **GADILKAR, Kedar
Ahmednagar 414003 (IN)**
• **MANOLIKAR, Mandar
560100 Bangalore, Karnataka (IN)**

(74) Representative: **Evonik Patent Association
c/o Evonik Industries AG
IP Management
Bau 1042A/PB 15
Paul-Baumann-Straße 1
45772 Marl (DE)**

(56) References cited:
**WO-A1-2014/139469      CN-A- 103 239 434
CN-A- 103 860 517      US-A1- 2005 158 381**

**Description**

**Field of the Invention**

The present invention relates to the field of pharmaceutical and nutraceutical formulations. More specifically, the present invention relates to effervescent formulations of ornithine aspartate as well as processes for their manufacture.

**Background**

In order to enable disintegration with sufficient intensity and generate enough effervescence to provide good taste and flavor, effervescent formulations must contain significant amounts of gas generating agents as well as acids. Effervescent formulations of ornithine aspartate containing such gas generating agents and acids, however, were found to be prone towards chemical degradation during manufacture and/or storage.

CN103860517A addresses this problem. The document discloses a method aimed at avoiding the formation of chemical impurities by employing melt granulation of sodium bicarbonate and polyethylene glycol (PEG 6000). The product obtained, thus, is mixed with granules of ornithine aspartate co-granulated with tartaric acid using a non-aqueous process beforehand. The process disclosed in CN103860517A, however, exhibits a number of disadvantages: Melt granulation is a relatively complex process necessitating use of specialized equipment. Further, the process does not allow manufacture of formulations with low levels of impurity. Additionally, the process cannot incorporate high contents of gas generating agents and acids, thus, merely yielding formulations with a less than desirable level of effervescence.

**Detailed Description**

The problem underlying the present invention, thus, resides in providing a simple process for the manufacture of chemically pure and stable formulations of ornithine aspartate with high levels of effervescence.

This problem is solved by the present invention providing a process for the manufacture of an effervescent formulation of ornithine aspartate comprising the following steps:

-    granulation of an ornithine aspartate-mix comprising ornithine aspartate and a gas generating component, thus yielding granules G,

-    mixing the components of a final mix comprising granules G and an acid component, thus yielding an effervescent formulation of ornithine aspartate.

According to the process of the present invention granulation of ornithine aspartate is performed in the presence of a gas generating component with no substantial amounts of acids present. Minor amounts of acids present during this step however, have no significant detrimental impact. A person of skill in the art, relying on impurity measurements and stability studies as described in the present specification, will be able to determine suitable maximal levels of acids that can be present during this step in addition to the gas generating component. In other embodiments granulation of the ornithine aspartate-mix comprising ornithine aspartate and a gas generating component, yielding granules G, is performed with a molar ratio R1 = n(total amount of substance of gas generating agents forming the gas generating component) / n(total amount of substance of pharmaceutically acceptable acids forming the acid component) selected from the following: R1 > 10, R1 > 20, R1 > 30, R1 > 100. In another embodiment of the present invention no pharmaceutically acceptable acids forming the acid component are present during this step. In yet another embodiment of the present invention no pharmaceutically acceptable acids are present during this step.

Similarly, according to the process of the present invention mixing the components of the final mix comprising granules G and an acid component, thus yielding an effervescent formulation of ornithine aspartate is performed with no substantial amounts of gas generating agents added in addition to the gas generating agents added with granules G.

Minor amounts of gas generating agents added in addition to the gas generating agents added with granules G during this step however, have no significant detrimental impact. A person of skill in the art, relying on impurity measurements and stability studies as described in the present specification, will be able to determine suitable maximal levels of gas generating agents that can be added in addition to the gas generating agents added with granules G during this step. In other embodiments mixing the components of the final mix comprising granules G and an acid component, thus yielding an effervescent formulation of ornithine aspartate is performed with a molar ratio R2 = n(total amount of substance of pharmaceutically acceptable acids forming the acid component) / n(total amount of substance of gas generating agents added in addition to the gas generating agents added with granules G during this step) selected from the following: R2 > 10, R2 > 20, R2 > 30, R2 > 100. In another embodiment of the present invention no gas generating agents forming the gas generating component are added in addition to the gas generating agents added with granules G during this step. In yet another embodiment of the present invention no gas generating agents are added in addition to the gas generating agents added with granules G during this step.

L-ornithine-L-aspartate is the salt of L-ornithine and L-aspartic acid. In healthy individuals fed with a proper diet, L-ornithine and L-aspartate are synthesized de novo in sufficient quantities, but in certain states of disease, as a result of tissue damage, organ insufficiency, excessive metabolic demand, growth, pregnancy, or deficiencies of urea cycle enzymes, it was found that supplementing these amino acids had beneficial effects. Both amino acids play key roles in ammonia detoxification and in proline and polyamine biosyntheses. Polyamines are considered critical for DNA synthesis and cell replication and have been shown to stimulate hepatic regeneration. Supplementation with ornithine in animal models demonstrated enhanced wound breaking strength and collagen deposition. It has been shown in vitro, in vivo and in perfused organs that urea synthesis from ammonia is limited by endogenous ornithine and that ornithine supplementation can promote urea formation to a significant degree. Low and high dose formulations of L-ornithine-L-aspartate are currently being marketed. Low dose formulations are used primarily as food supplements while high dose formulations (above 5 g) are used for example for lowering blood ammonia concentration and for eliminating symptoms of hepatic encephalopathy associated with liver cirrhosis. (Pol Merkur Lekarski. 2010 Jun; 28(168):490-5).

Effervescent formulations are intended to disintegrate fast, and rapidly and simultaneously release the active ingredients contained therein into an aqueous fluid. They comprise a mixture of ingredients (gas generating component and acid component) which release carbon dioxide upon contact with water. Effervescent formulations according to the present invention may further comprise additional pharmaceutically acceptable ingredients, such as excipients and coadjuvants selected from viscosity modifiers, fillers, disintegrants, lubricants, diluents, binders, glidants, antifoaming agents, wetting agents, colors, sweeteners and flavourings.

Disintegrants support rapid disintegration of tablets in aqueous fluids. Disintegrants increase the surface area of tablets in water rapidly disintegrating the tablet into small particles. Polymers which have a high degree of disintegration power include, inter alia, cross linked sodium carboxymethylcellulose, cross-linked hydroxypropylcellulose, high molecular weight hydroxypropylmethylcellulose, carboxymethylamide, potassium methacrylatedivinylbenzene copolymer, polymethylmethacrylate, cross-linked polyvinylpyrrolidone, high-molecular weight polyvinyl alcohols, microcrystalline cellulose, and the like. Particular examples of disintegrants are sodium starch glycolate, polymeric derivatives of acrylic acid, crosprovidone, and microcrystalline cellulose.

Fillers or diluents facilitate compression of powder and have an influence on the hardness of a tablet after compression. Furthermore they adjust the volume for potency. Such compounds comprise polyols, celluloses, starch and its derivatives, Lactose, isomalt, maltodextrin.

Lubricants are excipients which reduce inter-particle friction inside a tablet and reduce the reaction forces appearing on the die wall during compression or compaction. Lubricants are for example talcum, stearyl fumarate, polyethylene glycol, salts of benzoic acid, such as the sodium or lithium salt, L-leucine and magnesium stearate.

Flavouring agents (flavors) contribute to the taste for example the taste of a natural fruit, such as orange, lemon, apple, strawberry, vanilla, berries or of a herb, for example peppermint, or of broiled or fried meat, such as extracts from liver or yeast.

Sweetening agents are for example, saccharin, aspartame, cyclamate, sorbitol, sugar, polyols and mixtures thereof.

Colouring agents serve to give a pleasant appearance. Such agents are selected from any of the pharmaceutically or nutraceutically acceptable colors approved by regulatory agencies for example tartrazin (E102), crinoline yellow (E104), yellow orange (E110) and natural colors like anthocyanins.

Examples of binders are povidone, hydroxy propyl cellulose, carbomers, acrylic polymers, gums, PVA.

Examples of glidants are colloidal anhydrous silica, talc, L-leucine, stearates.

An example of an antifoaming agent is simitone.

An example of a wetting agent is polysorbate.

Effervescent formulations according to the present invention in the form of powders or granulates can be manufactured into numerous dosage forms including for example monolithic forms, such as tablets or pellets, as well as filled sachets. Tablet formulations comprising the effervescent formulations of the present invention may for example be formed by known compression pelleting techniques. In some cases dry densification processes may be used, e.g. briquetting, compression molding, and roller compaction.

In order to be administered the effervescent formulations of the present invention or dosage forms comprising the effervescent formulations of the present invention typically are dispersed in water or other aqueous fluids at room temperature, and administered orally. The amount of fluid is typically an amount that can conveniently be swallowed. For animals the formulations or dosage forms may be added to the food, or disintegrated in water and this form added to the food or injected into the mouth by means of a pipette.

The ornithine aspartate-mix granulated in the course of the process of the present invention comprises ornithine aspartate and a gas generating component. The product of this granulation are granules G. In addition to ornithine aspartate and the gas generating component the ornithine aspartate mix may comprise one or more of the following viscosity modifiers, fillers, disintegrants, lubricants, binders, antifoaming agents, wetting agents, colors, sweeteners and flavors.

According to the present invention Ornithine aspartate the salt of ornithine and aspartic acid is added to the ornithine aspartate mix. In the context of the present disclosure ornithine aspartate refers to L-ornithine L-aspartate, which is the salt of L-ornithine and L-aspartic acid.

The gas generating component according to the present invention consists of one or more gas generating agents. In the presence of the acid component and when contacted with water these gas generating agents release carbon dioxide. Accordingly, gas generating agents constituting the gas generating component are compounds releasing carbon dioxide when contacted with water in the presence of the acid component.

In other embodiments of the present invention the gas generating agents constituting the gas generating component are selected from the following: One or more carbonate salts, one or more bicarbonate salts, mixtures of one or more carbonate salts, mixtures of one or more bicarbonate salts, mixtures of one or more carbonate salts with one or more bicarbonate salts.

In other embodiments of the present invention the gas generating agents constituting the gas generating component are selected from the following: sodium carbonate, sodium bicarbonate, potassium carbonate, potassium bicarbonate, magnesium carbonate, calcium carbonate.

In other embodiments of the present invention the gas generating component consists of sodium bicarbonate.

In other embodiments of the present invention the total amount of gas generating agents constituting the gas generating component added to the formulation is sufficient to yield effervescent formulations of ornithine aspartate comprising 20 wt% to 40 wt% of gas generating component.

According to the present invention an ornithine aspartate-mix comprising ornithine aspartate and a gas generating component are granulated, thus yielding granules G,

Granulation of the ornithine aspartate-mix can be performed by any of several methods known in the art such as aqueous granulation, non-aqueous solvent based granulation, dry granulation, compaction, slugging, melt granulation, agglomeration by heat application and combinations thereof. Such methods for granulation are well established in the field and thus well known to a person of skill in the art. They have also been described in detail in the literature (cf. e.g. Aulton's Pharmaceutics: The Design and Manufacture of Medicines, Chapter: Pharmaceutical Technology of Granule production by Michael E. Aulton, Kevin M. G. Taylor, Part 5, pages 472-485; Handbook of Pharmaceutical, Granulation Technology, Executive Editor James Swarbrick, PharmaceuTech Inc. Pinehurst, North Carolina. 2005 by Taylor & Francis Group, LLC. Chapter, Theory Of granulation: An Engineering prospective pages 7-60).

In other embodiments of the present invention granulation conditions employed for granulation of the ornithine aspartate-mix are selected from the following: wet granulation, dry granulation, melt granulation.

In view of the fact that melt granulation is a relatively complex process necessitating use of specialized equipment, while the process of the present invention does not require melt granulation conditions to be used, in a specific embodiment of the present invention no melt granulation conditions are employed in the process.

According to the present invention the components of a final mix comprising granules G and an acid component, are mixed thus yielding an effervescent formulation of ornithine aspartate.

In addition to granules G and an acid component the final mix according to the present invention may comprise one or more of the following: Viscosity modifiers, fillers, disintegrants, lubricants, diluents, binders, glidants, antifoaming agents, wetting agents, colors, sweeteners and flavors.

Mixing of the final mix can be performed by any of several methods known in the art such as blending, high shear mixing, geometric mixing, tumbling, co-milling etc.. Such methods for mixing are well established in the field and thus well known to a person of skill in the art. They have also been described in detail in the literature (cf. e.g. Powder Technology, Handbook, Marcel Dekker, New York, 1997, Pages 43-56; Pharmaceutical Blending and Mixing ,1st edition, edited by P. J. Cullen, Rodolfo Roma Ãach, Nicolas Abatzaglou, Chris D. Rielly. Willey Publication. Chapter 6, Continuous Powder Mixing. Pg. 102-484).

In other embodiments of the present invention mixing conditions employed for mixing of the final mix are selected from the following: Blending, high shear mixing, geometric mixing.

The acid component according to the present invention consists of one or more organic or inorganic pharmaceutically acceptable acids. In the presence of the acid component and when contacted with water the gas generating agents release carbon dioxide. Accordingly, pharmaceutically acceptable acids constituting the acid component are compounds inducing release of carbon dioxide when contacted with water in the presence of the gas generating component.

In other embodiments of the present invention pharmaceutically acceptable acids constituting the acid component are selected from the following acids as well as partial salts of the following acids with alkaline or alkaline earth metals in the case of polybasic acids: citric acid, tartaric acid, malic acid, adipic acid, succinic acid, fumaric acid, ascorbic acid, maleic acid, mixtures thereof.

In other embodiments of the present invention the acid component consists of citric acid.

According to the present invention pharmaceutically acceptable acids constituting the acid component are added in overall molar excess to the gas generating components constituting the gas generating component. Typically, pharmaceutically acceptable acids constituting the acid component and gas generating agents constituting the gas generating component are added to the formulation in an amount yielding effervescent formulations with a molar ratio of acid component to gas generating component in the range of 7 : 1 to 1.1 : 1.

An advantage of the process of the present invention resides in the fact that it can be performed at high levels of humidity, including humidity levels of up to 50% relative humidity.

The present invention further comprises effervescent formulations of ornithine aspartate obtainable by a process according to the invention.

In addition to ornithine aspartate, gas generating component and acid component effervescent formulations according to the present invention may further comprise one or more excipients selected from the following: Viscosity modifiers, fillers, disintegrants, lubricants, diluents, binders, glidants, antifoaming agents, wetting agents, colors, sweeteners, flavors.

In a specific embodiment of the present invention effervescent formulations according to the present invention contain one or more additional pharmaceutically acceptable ingredients selected from: fillers, lubricants, diluents, binders, glidants, colors, sweeteners, flavors.

Typically, effervescent formulations according to the present invention contain ornithine aspartate, gas generating component, acid component and additional pharmaceutically acceptable ingredients in the following percentages (for each specific formulation percentages must be selected to add up to 100%):

> ornithine aspartate: 10% - 60%
> gas generating component: 10% - 40%
> acid component: 15% - 50%
> fillers/ diluents: 0% - 25%
> lubricants/glidants: 0% - 15%
> binders: 0% - 10%
> sweeteners: 0% - 10%
> flavourings: 0% - 2%

In a specific embodiment of the present invention effervescent formulations according to the present invention contain ornithine aspartate, gas generating component and acid component as well as the following pharmaceutically acceptable ingredients: Fillers, lubricants, diluents, binders, glidants, colors, sweeteners and flavors.

In another aspect the present invention provides a process for the manufacture of stable effervescent formulations of ornithine aspartate containing large amounts of gas generating agents. Formulations containing large amounts of gas generating agents are desirable for a number of applications because they are capable of imparting good palatability on drinks obtained therefrom by carbonation of the liquid and evolution of carbon dioxide in gaseous form. One reason for this effect is that dissolved carbon dioxide contributes to the taste directly by interacting with the sour taste buds. Furthermore, evolved carbon dioxide in gaseous form helps in improving the flavor and thus also contributes to the taste indirectly through olfactory sensing. Accordingly, the amount of carbon dioxide that can be produced by an effervescent formulation is an important factor and in the present context it is referred to as the gas generating capacity of an effervescent formulation. In order to impart beneficial taste properties to a drink obtained therefrom it is desirable to apply effervescent dosage forms exhibiting sufficient gas generating capacity to saturate the drink with carbon dioxide. In view of the fact that the typical volume of a drink obtained from an effervescent formulation is about 100 mL and, further, that 100 mL of water are capable of dissolving about 150 mg of carbon dioxide at room temperature (25 °C), a gas generating capacity of 150 mg carbon dioxide is usually sufficient to impart the corresponding beneficial taste properties.

Therefore, in one embodiment, the process of the present invention allows to obtain effervescent formulations of ornithine aspartate exhibiting a gas generating capacity of more than 150 mg carbon dioxide per gram of ornithine aspartate.

Accordingly, further, in one embodiments the present invention provides effervescent formulations of ornithine aspartate obtainable by a process of the present invention, exhibiting a gas generating capacity of more than 150 mg carbon dioxide per gram of ornithine aspartate.

As indicated above effervescent formulations according to the present invention in the form of powders or granulates can be manufactured into numerous dosage forms including for example monolithic forms, such as tablets or pellets, as well as filled sachets. Accordingly, in another embodiment the present invention comprises tablets, pellets or sachets comprising effervescent formulations of ornithine aspartate according to the present invention.

Description of Figures

Fig. 1     Flow chart of the manufacturing process
Fig. 2     Effervescence/ $CO_2$ generation characteristics of sample-$G_I$ in water (100mL) at 25°C
Fig. 3     Effervescence/ $CO_2$ generation characteristics of samples $H_I$, $J_I$, $N_I$ $O_C$, $P_I$, $Q_C$ in water (100mL) at 25°C
Fig. 4     Effervescence/ $CO_2$ generation characteristics of sample-$V_I$ in water (100mL) at 25°C

**Examples**

**(1) Analytical methodology**

The following section describes the analytical methods used for analyzing samples A to V below.

**Method for Impurity analysis:**

*Chromatographic Conditions*

| | |
|---|---|
| Column: | Waters Spherisorb Amino 5μ (250x4.6)mm |
| Mobile Phase: | Buffer: Acetonitrile (27.5:72.5) |
| | Buffer: 0.05M KH2PO4 |
| Wavelength: | 210 nm |
| Column Temp: | 25°C |
| Injection volume: | 100 μL |
| Flow rate: | 1.0 mL/minute |
| Run time: | 120 minutes |
| Sample Temp: | Ambient |
| Solvents used were of HPLC grade. | |
| Diluent: Mobile phase | |

*Standard preparation*

L-Ornithine Lactam Impurity ((3S)-3-Aminopiperidin-2-one*Hcl) solution: 5mg Impurity was weighed and transferred in100 mL volumetric flask. 30 mL water was added and sonicated to dissolve. Diluted up to the volume with water. (Approx. Concentration - 50 ppm)
Fumaric acid solution: 20mg of Fumaric acid was weighed and transferred in1000 mL volumetric flask. 300 mL water added and sonicated to dissolve. Diluted up to the volume with water. (Approx. Concentration - 20 ppm)
Malic acid solution: 150mg Malic acid was weighed and transferred in 100 mL volumetric flask. 30 mL water was added and sonicated to dissolve. Diluted up to the volume with water. (Approx. Concentration - 1500 ppm)
L-Arginine solution: 50mg L-Arginine was weighed and transferred in100 mL volumetric flask. 30 mL water was added and sonicated to dissolve. Diluted up to the volume with water. (Approx. Concentration - 500 ppm)

*System Suitability Solution Preparation:*

130mg of L-Ornithine L-Aspartate API was weighed and transferred in 50 mL volumetric flask. 2 mL of each of the impurity stock solutions were transferred to the flask and diluted to volume with diluent. (Approx. concentrations: Lactam - 2ppm, Fumaric acid - 0.8ppm, Malic acid - 60ppm, L-Arginine - 20ppm, L-Ornithine L-Aspartate - 2600 ppm)

*Sample preparation:*

For sachets containing effervescent granules, entire contents of five sachets were emptied and weighed to obtain average weight of sachet. For effervescent tablets, five tablets were weighed to obtain average weight of a tablet and then crushed in to powder. An amount of effervescent formulation to be analyzed equivalent to 1000 mg of L-Ornithine L-Aspartate was weighed and transferred in 100 mL volumetric flask. 30 mL water was added and effervescence allowed to go away. Sonicated to dissolve and diluted up to the mark with water. This was stock solution containing about 10000 ppm of L-Ornithine L-Aspartate. Further, transferred 3 mL of stock solution in to a 10 mL volumetric flask and diluted up to the mark with mobile phase (Approx. L-Ornithine L-Aspartate concentration - 3000ppm). Filtered the solution through 0.45μ Nylon filter. First few mL of filtrate was discarded. This was sample solution used for injection in chromatographic system.

*Placebo preparation:*

Placebo equivalent to 1000 mg of L-Ornithine L-Aspartate (Amount of Placebo = Average weight of sachet or tablet - L-Ornithine L-Aspartate content in each sachet or tablet (viz. 1000 mg)) was weighed and transferred in 100 mL volumetric flask. 30 mL water was added and effervescence allowed to go away. Sonicated to dissolve and diluted up to the mark with water. This was stock solution. Further, transferred 3 mL of stock solution in to a 10 mL volumetric flask and diluted up to the mark with mobile phase. Filtered the solution through 0.45μ Nylon filter. First few mL of filtrate was discarded. This was placebo solution used for injection in chromatographic system.

*Procedure:*

100 μl each of diluent, system suitability solution, Fumaric acid solution (0.8 ppm), and Placebo solution were injected into the chromatograph and the chromatograms were recorded. It was ensured that, system suitability parameters were fulfilled and there was no interference from the blank and placebo chromatograms at the retention time of the main peaks and impurity peaks.
The sample sequence for six batches typically followed was as given below. Same sequence was adopted for more than six batches.

| Sample name | No of injections | Type of testing |
|---|---|---|
| Diluent | 1 | Blank |
| System Suitability Solution | 3 | System suitability and known impurity standard |
| Sample solution | 1 | Sample preparation of sample I |
| Sample solution | 1 | Sample preparation of sample II |
| Sample solution | 1 | Sample preparation of sample III |
| Sample solution | 1 | Sample preparation of sample IV |
| Sample solution | 1 | Sample preparation of sample V |
| Sample solution | 1 | Sample preparation of sample VI |
| Placebo | 1 | To check placebo interference |
| Diluent | 1 | Blank |
| System Suitability Solution | 1 | System suitability and known impurity standard |

*System Suitability:*

The following system suitability criteria were measured form injection of System Suitability Solution:

Theoretical plates for L-Aspartic acid peak are not less than 2000
% RSD for area of Impurity peaks are not more than 3%.

*Calculation:*

% Individual Known Impurity in sample

$$\% \text{ Individual Known Impurity in sample} = \frac{A\,smp}{A\,std} \times \frac{\text{Wt std}}{\text{D stock}} \times \frac{2}{50} \times \frac{100}{\text{Wt}\,smp} \times \frac{50}{5} \times \frac{\text{Avg}}{\text{LC}} \times 100$$

Where:

A *smp:*    Area of Impurity obtained from measurement of sample solution

A *std:*    Average area of Impurity obtained from a series of measurements of system suitability solutions

Wt *std:*    Weight of Impurity standard in mg

Wt *smp:*    Weight of sample in mg

D stock:    Final dilution volume of impurity stock solution in mL

LC:    L-Ornithine L-Aspartate content in each sachet or tablet in mg

Avg:    Average weight of sachet or tablet in mg

Note: Calculation of known impurities was done using areas of individual impurity peaks as observed in system suitability solution.

% Unknown Impurity in sample

$$\% \ Unknown \ Impurity \ in \ sample = \frac{A \ smp}{A \ tot - A \ plc - A \ blk} \ x \ 100$$

Where:

A *smp:*    Area of Impurity in sample preparation

A *tot:*    Total area in sample chromatogram

A *plc*:    Area of placebo peaks in sample chromatogram

A *blk*:    Area of blank peaks in sample chromatogram

% Total Impurity in sample

% Individual Known Impurity + % Individual Unknown Impurity

Total Impurity in sample (in ppm)

% Total impurity x 10000

**Analytical methodology for determination of effervescence characteristics**

Samples were placed in glass beaker containing 100mL of demineralized water at a temperature of 25°C. The change in the total weight of contents was noted using a suitable digital balance over the period of 10minutes. Total time required for visual disappearance of effervescence was also noted. The amount of $CO_2$ dissolved in water was arithmetically calculated by subtracting the amount of gas evolved in 10 minutes from the theoretical gas generating capacity.

**(2) Formulation Details**

**List of Ingredients**

Following is a list of the Excipients, manufacturers and specification, used in the examples below (USP = United States Pharmacopeia, BP = British Pharmacopeia):

Table 1: List of ingredients used

| S.No. | Name of excipient | Manufacturer/Supplier | Specification |
|---|---|---|---|
| 1 | L-ornithine L-Aspartate | Evonik Industries AG, Germany | In House |
| 2 | Povidone (PVPK 25) | Koje Polymer | USP |
| 3 | Color FD &C 6 Alum Lake | Sensient | USP |
| 4 | Sodium bicarbonate | Merck inc. | USP |

(continued)

| S.No. | Name of excipient | Manufacturer/Supplier | Specification |
|---|---|---|---|
| 5 | Aspartame | Nutra Sweet. | USP |
| 6 | Flavor: Orange (501071) | Firmenich | USP |
| 7 | Citric acid Anhydrous | Sunil Pharma | BP |
| 8 | Polyethylene glycol (PEG 6000) | Sasol | USP |
| 9 | L-Leucine | Evonik Industries AG, Germany | In House |
| 10 | Isomalt (galen IQ 721) | Beneo-Palatinit gmbh | USP |

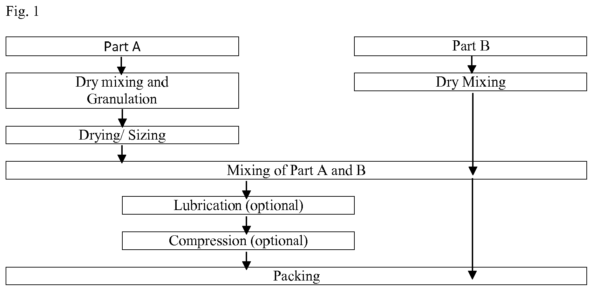

**A Flow chart of the manufacturing process is displayed in Fig. 1.**

**Oral formulations of L-Ornithine L-Aspartate**

Suffixes denote type of example: $_C$ = Comparative Example; $_I$ = inventive Example

Table 2: Formula for examples A-F

| Ingredients | Quantities | | | |
|---|---|---|---|---|
| | Example-A$_C$ | Example-B$_C$ | Example-C$_C$ | Example-D$_C$, E$_C$, F$_I$ |
| L-Ornithine L-Aspartate | 1000.0 | 1000.0 | 1000.0 | 1000.0 |
| Sodium bicarbonate | - | - | 950.00 | 950.00 |
| Citric Acid | - | 1525.0 | - | 1525.0 |
| Total weight | 1000.0 | 2525.0 | 1950.0 | 3475.0 |

Table 3: Formula for example G$_I$

| | Ingredients | Weight per dose (mg) |
|---|---|---|
| Part - A (L-Ornithine L-Aspartate + Carbonate Salt Granules) | L-Ornithine L-Aspartate | 1000.0 |
| | Povidone (PVP K 25) | 16.5 |
| | Color FD&C YELLOW 6 ALUM Lake | 6.5 |
| | Sodium Bicarbonate | 950.0 |
| | Weight of Granules | 1973.0 |
| Part- B (Acidifier in dry mix) | Aspartame | 90.0 |
| | Flavor: Orange 501071 | 50.0 |
| | Isomalt (galen IQ721) | 62.0 |
| | Citric Acid Anhydrous | 1525.0 |
| | Weight of dry mix | 1727.0 |
| TOTAL WEIGHT OF PART-A & B | | 3700.0 |

**Detailed method of preparation of samples**

Examples B$_C$&$_C$: L-Ornithine L-Aspartate was granulated using purified water, dried and sized to mix with either Citric acid (example B) or Sodium bicarbonate (example C)

Example $D_C$: L-Ornithine L-Aspartate was granulated along with Citric acid and Sodium bicarbonate using purified water, dried and sized.

Examples $E_C$&$F_I$: L-Ornithine L-Aspartate was granulated using purified water either with Citric acid (example-E) or Sodium bicarbonate (example-F) dried and sized. Such granules were further mixed with Sodium bicarbonate (example-E) or Citric acid (example-F).

Example $G_I$: L-Ornithine L-Aspartate, Povidone PVP K25, Color and Sodium bicarbonate were accurately weighed, mixed and granulated with water. Dried granules (Part- A) were sized and mixed with Aspartame, orange flavor, Isomalt and citric acid anhydrous (Part- B).

Resulting mixed granules were finally packed in HDPE bottles for stability studies carried out for the period of 12 days at 40°C/75%RH (relative humidity).

Table 4: Impurity results: Effect of method of incorporation of acidifier and gas generating agent on stability of L-Ornithine L-Aspartate granules (* -> Total impurities are expressed in parts per million(PPM))

| Sample | Qualitative Composition & process | Total Impurities (PPM)* | |
| --- | --- | --- | --- |
| | | Initial | 12days,40°C/ 75%RH |
| $A_C$ | Aqueous Granulation of L-Ornithine L-Aspartate | 50 | 50 |
| $B_C$ | Aqueous Granulation of L-Ornithine L-Aspartate Extra Granular addition of Citric acid Anhydrous | 360 | 930 |
| $C_C$ | Aqueous Granulation of L-Ornithine L-Aspartate Extra Granular addition of NaHCO3 | 110 | 270 |
| $D_C$ | Aqueous Granulation together of L-Ornithine L-Aspartate Sodium Bicarbonate and Citric acid Anhydrous | 150 | 470 |
| $E_C$ | Aqueous Granulation of L-Ornithine L-Aspartate and Citric acid Anhydrous Extra Granular addition of Sodium Bicarbonate | 80 | 1060 |
| $F_I$ | Aqueous Granulation of L-Ornithine L-Aspartate and Sodium Bicarbonate Extra Granular addition of Citric acid | 90 | 150 |
| $G_I$ | Aqueous Granulation of Part-A ingredients Extra Granular addition of Part-B ingredients | 60 | 170 |

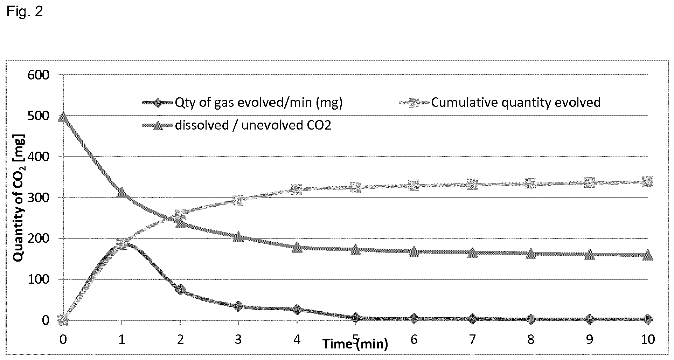

Remarks on impurities in examples $A_C$ to $G_I$: Impurities generated in inventive examples ($F_I$ to $G_I$) was lower than that of comparative examples ($B_C$ to $E_C$).
Sample-$G_I$ was analyzed for effervescence/ $CO_2$ generation characteristics in water (100mL) at 25 °C(cf. Fig. 2).

Table 5: Effervescence/ $CO_2$ generation characteristics of sample $G_I$

| Calculated $CO_2$ generation capacity (i) | Time for complete effervescence | Amount of $CO_2$ evolved in 10 minutes (ii) | Amount of $CO_2$ in solution after 10 min (i-ii) |
| --- | --- | --- | --- |
| 497mg | 4 min | 338mg | 160mg |

Considering that the solubility of Carbon dioxide in water at 25 ° C is about 1.45g / L (as reported in Wikipedia), sample-G could supersaturate the drink with carbon dioxide giving enough carbonation for imparting good taste.

Based on above data additional experimentation was planned to further study effect of process and amount of acidifier in the formulations by adopting above processes

Table 6: Examples $H_I$, $I_I$ and $J_I$ (inventive)

| | Ingredients | Weight per dose (mg) | | |
|---|---|---|---|---|
| | | Example-$H_I$ | Example-$I_I$ | Example-$J_I$ |
| Part - A (L-Ornithine L-Aspartate + Carbonate salt Granules) | L-Ornithine L-Aspartate | 1000.0 | 1000.0 | 1000.0 |
| | Povidone (PVP K 25) | 16.5 | 16.5 | 16.5 |
| | Color FD&C YELLOW 6 ALUM Lake | 6.5 | 6.5 | 6.5 |
| | Sodium Bicarbonate | 950.0 | 950.0 | 950.0 |
| | Weight of Granules | 1973.0 | 1973.0 | 1973.0 |
| Part- B (Acidifier in dry mix) | Aspartame | 90.0 | 90.0 | 90.0 |
| | Flavor: Orange 501071 | 50.0 | 50.0 | 50.0 |
| | Isomalt (galen IQ721) | 62.0 | 62.0 | 62.0 |
| | Citric Acid Anhydrous | 1525.0 | 724.4 | 344.0 |
| | Weight of dry mix | 1727.0 | 926.4 | 546.0 |
| TOTAL WEIGHT OF PART-A & B | | 3700.0 | 2899.4 | 2519.0 |

Detailed method of preparation for examples-$H_I$, $I_I$ and $J_I$

Part-A: L-Ornithine L-Aspartate, Povidone PVP K25, Color and Sodium bicarbonate were accurately weighed, mixed and granulated with water in a rapid mixer granulator. Granules were dried in a fluidized bed processor to the LOD of <1%. Dried granules were sized to get #25 ASTM passing granules.

Part-B: Aspartame, flavor, Isomalt and Citric acid anhydrous were passed thru #25 ASTM sieve.

Part-A and B were mixed together for 5minutes.

Table 7: Formula for examples $K_C$, $L_C$ and $M_C$ (Comparative)

| | Ingredients | Weight per dose (mg) | | |
|---|---|---|---|---|
| | | Example-$K_C$ | Example-$L_C$ | Example-$M_C$ |
| Part - A (L-Ornithine L-Aspartate + acidifier Granules) | L-Ornithine L-Aspartate | 1000.0 | 1000.0 | 1000.0 |
| | Povidone (PVP K 25) | 16.5 | 16.5 | 16.5 |
| | Color FD&C YELLOW 6 ALUM Lake | 6.5 | 6.5 | 6.5 |
| | Citric Acid Anhydrous | 1525.0 | 724.4 | 344.0 |
| | Weight of Granules | 2548.0 | 1747.4 | 1367.0 |
| Part- B (Carbonate salt in dry mix) | Aspartame | 90.0 | 90.0 | 90.0 |
| | Flavor: Orange 501071 | 50.0 | 50.0 | 50.0 |
| | Isomalt (galen IQ721) | 62.0 | 62.0 | 62.0 |
| | Sodium Bicarbonate | 950.0 | 950.0 | 950.0 |
| | Weight of dry mix | 1152.0 | 1152.0 | 1152.0 |
| TOTAL WEIGHT OF PART-A & B | | 3700.0 | 2899.4 | 2519.0 |

Detailed method of preparation for examples-$K_C$, $L_C$ and $M_C$

Part-A: L-Ornithine L-Aspartate, Povidone PVP K25, Color and Citric acid were accurately weighed, mixed and granulated with water in a rapid mixer granulator. Granules were dried in a fluidized bed processor to the LOD of <1 %. Dried granules were sized to get #25 ASTM passing granules.

Part-B: Aspartame, flavor, Isomalt and Sodium bicarbonate were passed thru #25 ASTM sieve.

Part-A and B were mixed together for 5minutes.

These samples (H to M) were tested for impurity generation on storage. The results are mentioned in table 8 below.

Table 8: Impurity data for samples H to M

| Sample | Total Impurities (PPM)* | |
|---|---|---|
| | Initial | 12days,40°C/75%RH |
| $H_I$ | 90 | 130 |
| $I_I$ | 70 | 110 |
| $J_I$ | 100 | 110 |
| $K_C$ | 70 | 1500 |
| $L_C$ | 60 | 2560 |
| $M_C$ | 70 | 3270 |

Remarks on impurities in examples $H_I$ to $M_C$: Impurities generated in inventive examples ($H_I$ to $J_I$) were significantly lower than those of comparative examples ($K_C$ to $M_C$). This signifies the importance of method of addition of the acidic and gas generating components.

Based on above results, additional experiments ($N_I$, $O_C$ and $P_I$) were planned to evaluate effervescence characteristics using different inherent gas generation capacities of the formulations. Additionally example $Q_C$ was planned to study comparative properties of the prior art (CN103860517)

Table 9: Examples $N_I$, $O_C$ and $P_I$

| | Ingredients | Weight per dose (mg) | | |
|---|---|---|---|---|
| | | Example- $N_I$ | Example-$O_C$ | Example- $P_I$ |
| Part - A (L-Ornithine L-Aspartate + Carbonate salt Granules) | L-Ornithine L-Aspartate | 1000.0 | 1000.0 | 700 |
| | Povidone (PVP K 25) | 16.5 | 16.5 | 11.55 |
| | Color FD&C YELLOW | 6.5 | 6.5 | 4.55 |
| | Sodium Bicarbonate | 950.0 | 950.0 | 665.0 |
| | Weight of Granules | 1973.0 | 1973.0 | 1381.1 |
| Part- B (Acidifier in dry mix) | Aspartame | 90.0 | 90.0 | 90.0 |
| | Flavor: Orange 501071 | 50.0 | 50.0 | 50.0 |
| | Isomalt (galen IQ721) | 62.0 | 62.0 | 62.0 |
| | Citric Acid Anhydrous | 1525.0 | 218.0 | 1070.0 |
| | Weight of dry mix | 1727.0 | 420.0 | 1272.0 |

(continued)

|  | Ingredients | Weight per dose (mg) | | |
|---|---|---|---|---|
|  |  | Example- $N_I$ | Example- $O_C$ | Example- $P_I$ |
| Lubricants for tabletting | Polyethylene Glycol 6000 | 50.0 | - | - |
|  | L-Leucine | 50.0 | - | - |
| TOTAL WEIGHT OF PART-A & B | | 3800.0 | 2393.0 | 3653.1 |

Detailed method of preparation for examples-$N_I$, $O_C$, $P_I$

Part-A: L-Ornithine L-Aspartate, Povidone PVP K25, Color and Sodium bicarbonate were accurately weighed, mixed and granulated with water in a rapid mixer granulator. Granules were dried in a fluidized bed processor to the LOD of <1%. Dried granules were sized to get #25 ASTM passing granules.

Part-B: Aspartame, flavor, Isomalt and Citric acid anhydrous were passed thru #25 ASTM sieve.

Part-A and B were mixed together for 5minutes.

In case of example- N; part-A, part-B, PEG 6000 and L-Leucine were mixed together and compressed into tablets using 25mm tablet tooling.

Table 10: Example $Q_C$ (As per CN103860517)

|  | Ingredients | Weight per dose (mg) |
|---|---|---|
|  |  | Example Q |
| Part - A (L-Ornithine L-Aspartate + tartaric acid granules) | L-Ornithine L-Aspartate | 1000.0 |
|  | Kollidon VA 64 | 45.0 |
|  | Tartaric acid | 338.0 |
|  | Ethanol (q.s. to 8% Povidone solution) | Q.S |
|  | Weight of Granules | 1383.0 |
| Part- B (Carbonate + PEG granulate) | Sodium Bicarbonate | 210.0 |
|  | PEG 6000 | 130.0 |
|  | Weight of melt granulates | 340.0 |
| Dry excipients | Aspartame | 0.75 |
|  | Flavor | 0.75 |
| TOTAL WEIGHT OF PART-A, B &dry excipients | | 1724.5 |

Detailed method of preparation for example-$Q_C$

Part-A: L-Ornithine L-Aspartate and tartaric acid mixed together. Kollidon VA 64 dissolved in Ethanol to give 8% solution. This solution was used to granulate powder mixture and dried. Dried granules were sized to get #25 ASTM passing granules.

Part-B: PEG 6000 was mixed with sodium bicarbonate and melt granulated at 60 deg C in water bath. Cooled and illed to get were passed thru #25 ASTM sieve.

Part-A and B were mixed together for 5minutes.

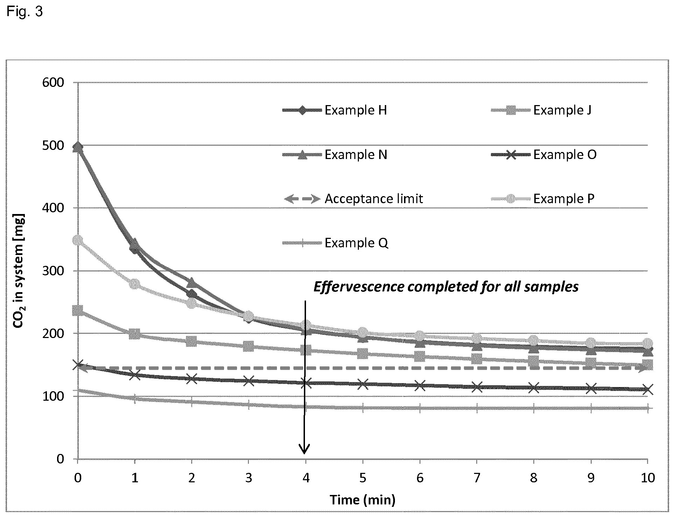

Samples $H_I$, $J_I$, $N_I$ $O_C$, $P_I$, $Q_C$ were analyzed for effervescence/ $CO_2$ generation characteristics in water (100mL) at 25°C (cf. Fig. 3).

Table 11: Effervescence/ $CO_2$ generation characteristics of samples in water (100mL) at 25 °C

| Sample | Calculated $CO_2$ generation capacity (i) | Time for complete effervescence | Amount of $CO_2$ evolved in 10 min (ii) | Amount of $CO_2$ in solution after 10 min (i-ii) |
|---|---|---|---|---|
| $Q_C$ | 110mg | 2min | 29mg | 81mg |
| $O_C$ | 150mg | 2min | 39mg | 111mg |
| $J_I$ | 236mg | 4min | 86mg | 150mg |
| $P_I$ | 348mg | 4min | 164mg | 184mg |
| $H_I$ | 497mg | 4min | 122mg | 176mg |
| $N_I$ | 497mg | 4min | 326mg | 172mg |

Remarks on effervescence characteristics in examples $H_I$, $J_I$ and $N_I$ to $Q_C$ : Considering that the solubility of Carbon dioxide in water at 25 ° C is about 1.45g / L (as reported in Wikipedia), all samples excluding those of the comparative examples ($O_C$ and $Q_C$) could supersaturate the drink with carbon dioxide giving enough carbonation for imparting good taste.

The impurity generation results for examples $H_I$, $N_I$ and $Q_C$ are in table below.

Table 12: Impurity data for samples $H_I$, $N_I$ and $Q_C$

| Sample | Total Impurities (PPM)* | |
|---|---|---|
| | Initial | 12days,40°C/75%RH |
| $H_I$ | 90 | 130 |
| $N_I$ | 50 | 60 |
| $Q_C$ | 100 | 3090 |

Remarks on impurities in examples $H_I$, $N_I$ and $Q_C$: Impurities generated in inventive examples ($H_I$ and $N_I$) were significantly lower than those of comparative example from prior art ($Q_C$). This signifies the importance of method of addition of the acidic and gas generating components.

Additional examples were studied to ascertain the effect of change in type of acid on impurity generation behavior of the invention.

Table 13: Example $R_I$ (replacement of Citric acid with tartaric acid) Positive example

| | Ingredients | Weight per dose (mg) |
|---|---|---|
| | | Example- $R_I$ |
| | L-Ornithine L-Aspartate | 1000.0 |
| | Povidone (PVP K 25) | 16.5 |
| | Color FD&C YELLOW | 6.5 |
| | Sodium Bicarbonate | 950.0 |
| Part - A (L-Ornithine L-Aspartate + Carbonate salt Granules) | Weight of Granules | 1973.0 |

(continued)

|  | Ingredients | Weight per dose (mg) |
|---|---|---|
|  |  | Example- $R_I$ |
| Part- B (Acidifier in dry mix) | Aspartame | 90.0 |
|  | Flavor: Orange 501071 | 50.0 |
|  | Isomalt (galen IQ721) | 62.0 |
|  | Tartaric Acid | 1525.0 |
|  | Weight of dry mix | 1727.0 |
| TOTAL WEIGHT OF PART-A & B | | 3700.0 |

Detailed method of preparation for example- $R_I$

Part-A: L-Ornithine L-Aspartate, Povidone PVP K25, Color and Sodium bicarbonate were accurately weighed, mixed and granulated with water in a rapid mixer granulator. Granules were dried in a fluidized bed processor to the LOD of <1%. Dried granules were sized to get #25 ASTM passing granules.

Part-B: Aspartame, flavor, Isomalt and Tartaric acid were passed thru #25 ASTM sieve.

Part-A and B were mixed together for 5minutes.

Table 14: Formula for example Sc (Comparative against example $R_I$)

|  | Ingredients | Weight per dose (mg) |
|---|---|---|
|  |  | Example- $S_C$ |
| Part - A (L-Ornithine L-Aspartate + acidifier Granules) | L-Ornithine L-Aspartate | 1000.0 |
|  | Povidone (PVP K 25) | 16.5 |
|  | Color FD&C YELLOW 6 ALUM Lake | 6.5 |
|  | Tartaric Acid | 1525.0 |
|  | Weight of Granules | 2548.0 |
| Part- B (Carbonate salt in dry mix) | Aspartame | 90.0 |
|  | Flavor: Orange 501071 | 50.0 |
|  | Isomalt (galen IQ721) | 62.0 |
|  | Sodium Bicarbonate | 950.0 |
|  | Weight of dry mix | 1152.0 |
| TOTAL WEIGHT OF PART-A & B | | 3700.0 |

Detailed method of preparation for example- $S_C$

Part-A: L-Ornithine L-Aspartate, Povidone PVP K25, Color and Tartaric acid were accurately weighed, mixed and granulated with water in a rapid mixer granulator. Granules were dried in a fluidized bed processor to the LOD of <1%. Dried granules were sized to get #25 ASTM passing granules.

Part-B: Aspartame, flavor, Isomalt and Sodium bicarbonate were passed thru #25 ASTM sieve.

Part-A and B were mixed together for 5minutes.

Table 15: Impurity data for samples $R_I$ and $S_C$

| Results of impurity analysis of examples $R_I$ and $S_C$ Sample | Total Impurities (PPM)* | |
|---|---|---|
| | Initial | 12days,40°C/75%RH |
| $R_I$ | 150 | 780 |
| $S_C$ | 210 | 14470 |

Remarks on impurities in examples $R_I$ and $S_C$: Impurities generated in inventive example ($R_I$) was significantly lower than that of comparative examples ($S_C$). Thus as expected, in spite of change in type of acid, the impurity generation behavior of the formulations remain unaffected.

In order to study applicability of the invention to the type of granulation techniques, additional experiments ($T_I$ and $U_I$) were planned. Effect of melt granulation and dry granulation was studied.

Table 16: Examples $T_I$ and $U_I$ (inventive, demonstrating dry and melt granulation techniques)

| | Ingredients | Weight per dose (mg) | |
|---|---|---|---|
| | | Example-$T_I$ | Example- $U_I$ |
| | | Dry Granulation | Melt granulation |
| Part - A (L-Ornithine L-Aspartate + Carbonate salt Granules) | L-Ornithine L-Aspartate | 1000.0 | 1000.0 |
| | Povidone (PVP K 25) | 16.5 | --- |
| | PEG 6000 | --- | 200.0 |
| | Color FD&C YELLOW | 6.5 | 6.5 |
| | Sodium Bicarbonate | 950.0 | 950.0 |
| | Weight of Granules | 1973.0 | 2156.5 |
| Part- B (Acidifier in dry mix) | Aspartame | 90.0 | 90.0 |
| | Flavor: Orange 501071 | 50.0 | 50.0 |
| | Isomalt (galen IQ721) | 62.0 | 62.0 |
| | Citric Acid Anhydrous | 1525.0 | 1525.0 |
| | Weight of dry mix | 1727.0 | 1727.0 |
| TOTAL WEIGHT OF PART-A & B | | 3700.0 | 3883.5 |

Detailed method of preparation

Dry Granulation (Example $T_I$):

Part-A: L-Ornithine L-Aspartate, Povidone PVP K25, Color and Sodium bicarbonate were accurately weighed, passed through # 40 ASTM sieve and mixed for 5 minutes. Resulting blend was compacted on to obtain compacts. The compacts were milled and sized through #25 ASTM sieve.

Part-B: Aspartame, flavor, Isomalt and Citric acid anhydrous were passed thru #25 ASTM sieve.

Part-A and B were mixed together for 5minutes.

Melt Granulation (Example U$_I$):

Part-A: L-Ornithine L-Aspartate, PEG 6000, colour and Sodium bicarbonate were accurately weighed and passed through # 40 ASTM sieve and mixed. Resulting blend mix was transferred to glass beaker and heated at 65°C on hot plate and mixed to ensure homogeneous mix. After complete melting of PEG 6000 melted mass was removed, cooled and sifted through # 25 ASTM sieve.

Part-B: Aspartame, flavor, Isomalt and Citric acid anhydrous were passed thru #25 ASTM sieve.

Part-A and B were mixed together for 5minutes.

Samples, T$_I$ and U$_I$ were tested for impurity generation on storage. The results are in table below.

Table 17: Impurity data for samples T$_I$ and U$_I$

| Sample | Total Impurities (PPM)* | |
|---|---|---|
| | Initial | 12days,40°C/75%RH |
| T$_I$ | 70 | 290 |
| U$_I$ | 40 | 100 |

Remarks on impurities in examples T$_I$ and U$_I$: Impurities generated in both the inventive examples (T$_I$ and U$_I$) was not increased significantly and the behavior was similar to other inventive examples mentioned earlier. Thus, in spite of change in type of granulation technique, the impurity generation behavior of the formulations remain unaffected.

Another experiment (sample V$_I$) explained below was planned by using ascorbic acid as acidic component and Elderberry extract as color and flavor.

Table 18: Formula for example V$_I$

| | Ingredients | Weight per dose (mg) |
|---|---|---|
| | | Example- V$_I$ |
| Part - A (L-ornithine L-aspartate + Carbonate salt Granules) | L-Ornithine L-Aspartate | 1000.0 |
| | Sodium Bicarbonate | 700.0 |
| | Weight of Granules | 1700.0 |
| Part - B (Acidifier in dry mix) | Aspartame | 180.0 |
| | Elderberry extract | 400 |
| | Isomalt (galen IQ721) | 1720 |
| | Ascorbic acid | 2000 |
| | Weight of dry mix | 6000.0 |

Detailed method of preparation for example-V

Part-A: L-Ornithine L-Aspartate and Sodium bicarbonate were accurately weighed, mixed and granulated with water in a rapid mixer granulator. Granules were dried in a fluidized bed processor to the LOD of <1 %. Dried granules were sized to get #25 ASTM passing granules.

Part-B: Aspartame, Elderberry extract, Isomalt and Ascorbic acid were passed thru #30 ASTM sieve.

Part-A and B were mixed together for 5minutes.

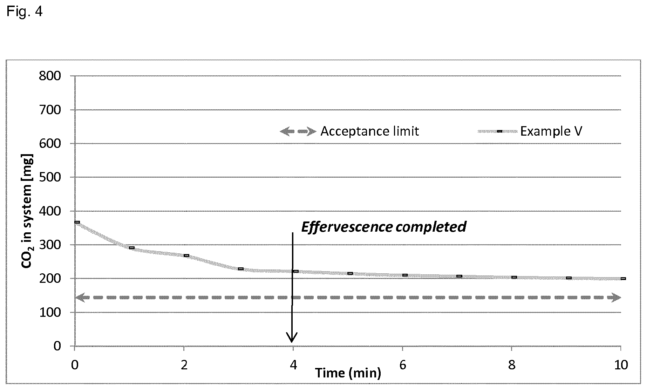

Sample-$V_I$ was analyzed for effervescence/ $CO_2$ generation characteristics in water (100mL) at 25 ° C (cf. Fig. 4).

Table 19: Effervescence/ $CO_2$ generation characteristics of sample V in water (100mL) at 25 ° C

| Sample | Calculated $CO_2$ generation capacity (i) | Time for complete effervescence | Amount of $CO_2$ evolved in 10 min (ii) | Amount of $CO_2$ in solution after 10 min (i-ii) |
|---|---|---|---|---|
| V | 367mg | 4min | 168mg | 199mg |

Remarks on effervescence characteristics in example $V_I$: As observed with other inventive examples, example $V_I$ exhibited acceptable effervescence generating characteristics. No significant effect of change in acidic component, color or flavor was observed.

**Claims**

1.  Process for the manufacture of an effervescent formulation of ornithine aspartate comprising the following steps:

    - granulation of an ornithine aspartate-mix comprising ornithine aspartate and a gas generating component, thus yielding granules G,
    - mixing the components of a final mix comprising granules G and an acid component, thus yielding an effervescent formulation of ornithine aspartate.

2.  Process according to claim 1, wherein granulation of the ornithine aspartate mix is performed under granulation conditions selected from the following: wet granulation, dry granulation, melt granulation.

3.  Process according to any one of claims 1 to 2, wherein the total amount of gas generating agents constituting the gas generating component added to the formulation is sufficient to yield effervescent formulations comprising 20 wt% to 40 wt% of gas generating component.

4.  Process according to any one of claims 1 to 3, wherein the one or more gas generating agents constituting the gas generating component are selected from the following: One or more carbonate salts, one or more bicarbonate salts, mixtures of one or more carbonate salts, mixtures of one or more bicarbonate salts, mixtures of one or more carbonate salts with one or more bicarbonate salts.

5.  Process according to any one of claims 1 to 4, wherein the one or more gas generating agents constituting the gas generating component are selected from the following: sodium carbonate, sodium bicarbonate, potassium carbonate, potassium bicarbonate, magnesium carbonate, calcium carbonate.

6.  Process according to any one of claims 1 to 5, wherein the gas generating component consists of sodium bicarbonate.

7.  Process according to any one of claims 1 to 6, wherein the pharmaceutically acceptable acids constituting the acid component are selected from the following acids as well as their partial salts with alkaline or alkaline earth metals in the case of polybasic acids: citric acid, tartaric acid, malic acid, adipic acid, succinic acid, fumaric acid, ascorbic acid, maleic acid, mixtures thereof.

8.  Process according to any one of claims 1 to 7, wherein the acid component consists of citric acid.

9.  Effervescent formulations of ornithine aspartate obtainable by a process according to any one of claims 1 to 8.

10. Effervescent formulations of ornithine aspartate according to claim 9, comprising one or more excipients selected from the following: Viscosity modifiers, fillers, disintegrants, lubricants, diluents, binders, glidants, antifoaming agents, wetting agents, colors, sweeteners, flavors.

**11.** Effervescent formulations of ornithine aspartate according to any one of claims 9 to 10, exhibiting a gas generating capacity of more than 150 mg carbon dioxide per gram of ornithine aspartate.

**12.** Tablet, pellet or sachet comprising an effervescent formulation of ornithine aspartate according to any one of claims 9 to 11.


**Patentansprüche**

**1.** Verfahren zur Herstellung einer Brauseformulierung von Ornithinaspartat, umfassend die folgenden Schritte:

- Granulieren eines Ornithinaspartatgemischs, das Ornithinaspartat und eine gaserzeugende Komponente umfasst, um ein Granulat G zu ergeben,
- Mischen der Komponenten eines Endgemischs, das das Granulat G und eine Säurekomponente umfasst, um eine Brauseformulierung von Ornithinaspartat zu ergeben.

**2.** Verfahren nach Anspruch 1, wobei das Granulieren des Ornithinaspartatgemischs unter Granulationsbedingungen durchgeführt wird, die ausgewählt sind aus den folgenden: Nassgranulation, Trockengranulation, Schmelzgranulation.

**3.** Verfahren nach einem der Ansprüche 1 bis 2, wobei die Gesamtmenge an gaserzeugenden Mitteln, die die der Formulierung zugegebene gaserzeugende Komponente bilden, ausreicht, um Brauseformulierungen zu ergeben, die 20 Gew.-% bis 40 Gew.-% an gaserzeugender Komponente umfassen.

**4.** Verfahren nach einem der Ansprüche 1 bis 3, wobei das eine oder die mehreren gaserzeugenden Mittel, die die gaserzeugende Komponente bilden, ausgewählt sind aus den folgenden: einem oder mehreren Carbonatsalzen, einem oder mehreren Bicarbonatsalzen, Gemischen von einem oder mehreren Carbonatsalzen, Gemischen von einem oder mehreren Bicarbonatsalzen, Gemischen von einem oder mehreren Carbonatsalzen mit einem oder mehreren Bicarbonatsalzen.

**5.** Verfahren nach einem der Ansprüche 1 bis 4, wobei das eine oder die mehreren gaserzeugenden Mittel, die die gaserzeugende Komponente bilden, ausgewählt sind aus den folgenden: Natriumcarbonat, Natriumbicarbonat, Kaliumcarbonat, Kaliumbicarbonat, Magnesiumcarbonat, Calciumcarbonat.

**6.** Verfahren nach einem der Ansprüche 1 bis 5, wobei die gaserzeugende Komponente aus Natriumbicarbonat besteht.

**7.** Verfahren nach einem der Ansprüche 1 bis 6, wobei die pharmazeutisch verträglichen Säuren, die die Säurekomponente bilden, ausgewählt sind aus den folgenden Säuren und ihren Partialsalzen mit Alkali- oder Erdalkalimetallen im Fall von mehrbasigen Säuren: Citronensäure, Weinsäure, Apfelsäure, Adipinsäure, Bernsteinsäure, Fumarsäure, Ascorbinsäure, Maleinsäure, Gemischen davon.

**8.** Verfahren nach einem der Ansprüche 1 bis 7, wobei die Säurekomponente aus Citronensäure besteht.

**9.** Brauseformulierungen von Ornithinaspartat, erhältlich durch ein Verfahren nach einem der Ansprüche 1 bis 8.

**10.** Brauseformulierungen von Ornithinaspartat nach Anspruch 9, umfassend einen oder mehrere Hilfsstoffe ausgewählt aus den folgenden: Viskositätsmodifikatoren, Füllstoffen, Sprengmitteln, Schmiermitteln, Verdünnungsmitteln, Bindemitteln, Gleitmitteln, Entschäumern, Netzmitteln, Farbstoffen, Süßungsmitteln, Aromastoffen.

**11.** Brauseformulierungen von Ornithinaspartat nach einem der Ansprüche 9 bis 10, die eine Gaserzeugungskapazität von mehr als 150 mg Kohlendioxid pro Gramm Ornithinaspartat zeigen.

**12.** Tablette, Pellet oder Sachet, umfassend eine Brauseformulierung von Ornithinaspartat nach einem der Ansprüche 9 bis 11.

**Revendications**

1. Procédé pour la fabrication d'une formulation effervescente d'aspartate d'ornithine comprenant les étapes suivantes :

   - granulation d'un mélange d'aspartate d'ornithine comprenant de l'aspartate d'ornithine et un composant de génération de gaz, fournissant ainsi des granules G,
   - mélange des composants d'un mélange final comprenant des granules G et un composant de type acide, fournissant ainsi une formulation effervescente d'aspartate d'ornithine.

2. Procédé selon la revendication 1, la granulation du mélange d'aspartate d'ornithine étant réalisée dans des conditions de granulation choisies parmi les suivantes : granulation humide, granulation sèche, granulation par fusion.

3. Procédé selon l'une quelconque des revendications 1 et 2, la quantité totale d'agents de génération de gaz constituant le composant de génération de gaz ajouté à la formulation étant suffisante pour fournir des formulations effervescentes comprenant de 20 % en poids à 40 % en poids de composant de génération de gaz.

4. Procédé selon l'une quelconque des revendications 1 à 3, l'agent ou les agents de génération de gaz constituant le composant de génération de gaz étant choisis parmi les suivants : un ou plusieurs sels de carbonate, un ou plusieurs sels de bicarbonate, des mélanges d'un ou plusieurs sels de carbonate, des mélanges d'un ou plusieurs sels de bicarbonate, des mélanges d'un ou plusieurs sels de carbonate avec un ou plusieurs sels de bicarbonate.

5. Procédé selon l'une quelconque des revendications 1 à 4, l'agent ou les agents de génération de gaz constituant le composant de génération de gaz étant choisis parmi les suivants : carbonate de sodium, bicarbonate de sodium, carbonate de potassium, bicarbonate de potassium, carbonate de magnésium, carbonate de calcium.

6. Procédé selon l'une quelconque des revendications 1 à 5, le composant de génération de gaz étant constitué de bicarbonate de sodium.

7. Procédé selon l'une quelconque des revendications 1 à 6, les acides pharmaceutiquement acceptables constituant le composant de type acide étant choisis parmi les acides suivants ainsi que leurs sels partiels avec des métaux alcalins ou alcalino-terreux dans le cas d'acides polybasiques : acide citrique, acide tartrique, acide malique, acide adipique, acide succinique, acide fumarique, acide ascorbique, acide maléique, des mélanges correspondants.

8. Procédé selon l'une quelconque des revendications 1 à 7, le composant de type acide étant constitué d'acide citrique.

9. Formulations effervescentes d'aspartate d'ornithine pouvant être obtenues par un procédé selon l'une quelconque des revendications 1 à 8.

10. Formulations effervescentes d'aspartate d'ornithine selon la revendication 9, comprenant un ou plusieurs excipients choisis parmi les suivants : modificateurs de viscosité, charges, désintégrants, lubrifiants, diluants, liants, agents glissants, agents antimousses, agents mouillants, colorants, édulcorants, arômes.

11. Formulations effervescentes d'aspartate d'ornithine selon l'une quelconque des revendications 9 et 10, présentant une capacité de génération de gaz de plus de 150 mg de dioxyde de carbone par gramme d'aspartate ornithine.

12. Comprimé, pastille ou sachet comprenant une formulation effervescente d'aspartate d'ornithine selon l'une quelconque des revendications 9 à 11.

Fig. 1

```
┌─────────────────────────┐              ┌─────────────────────────┐
│         Part A          │              │         Part B          │
└─────────────────────────┘              └─────────────────────────┘
             │                                        │
             ▼                                        ▼
┌─────────────────────────┐              ┌─────────────────────────┐
│     Dry mixing and      │              │       Dry Mixing        │
│       Granulation       │              │                         │
└─────────────────────────┘              └─────────────────────────┘
             │                                        │
             ▼                                        │
┌─────────────────────────┐                          │
│      Drying/ Sizing     │                          │
└─────────────────────────┘                          │
             │                                        │
             ▼                                        │
┌──────────────────────────────────────────────────────────────────┐
│                   Mixing of Part A and B                    ▼      │
└──────────────────────────────────────────────────────────────────┘
             │                                        │
             ▼                                        │
   ┌─────────────────────────────┐                    │
   │    Lubrication (optional)   │                    │
   └─────────────────────────────┘                    │
             │                                        │
             ▼                                        │
   ┌─────────────────────────────┐                    │
   │    Compression (optional)   │                    │
   └─────────────────────────────┘                    │
             │                                        │
             ▼                                        ▼
┌──────────────────────────────────────────────────────────────────┐
│                           Packing                                 │
└──────────────────────────────────────────────────────────────────┘
```

Fig. 2

Fig. 3

Fig. 4

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 103860517 A

- CN 103860517

**Non-patent literature cited in the description**

- *Pol Merkur Lekarski,* June 2010, vol. 28 (168), 490-5
- **MICHAEL E. AULTON ; KEVIN M. G. TAYLOR.** Aulton's Pharmaceutics: The Design and Manufacture of Medicines. 472-485

- PharmaceuTech Inc. Pinehurst. Handbook of Pharmaceutical, Granulation Technology. Taylor & Francis Group, LLC, 2005, 7-60
- Powder Technology, Handbook. Marcel Dekker, 1997, 43-56
- Pharmaceutical Blending and Mixing. 102-484